(19) Europäisches Patentamt
European Patent Office
Office européen des brevets

(11) Veröffentlichungsnummer: **0 043 348**
**A1**

(12) **EUROPÄISCHE PATENTANMELDUNG**

(21) Anmeldenummer: **81810259.2**

(22) Anmeldetag: **22.06.81**

(51) Int. Cl.³: **C 07 D 233/80**
**A 01 N 43/50**

(30) Priorität: **27.06.80 CH 4964/80**

(43) Veröffentlichungstag der Anmeldung:
**06.01.82 Patentblatt 82/1**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Hubele, Adolf, Dr.**
**Obere Egg 9**
**CH-4465 Magden(CH)**

(54) **Imidazolidin-2,4-dion-Derivate, Verfahren zu deren Herstellung und deren Verwendung als Mikrobizide.**

(57) Es werden neue Verbindungen der hierin definierten Formel I

```
                    O        O
   Cl               ‖        ‖
     \             C - N - CS-R
      ●=●        /      |
    ●     ●-N           |
      ●=●        \      |
     /             C - CH2          (1),
   Cl              ‖
                    O
```

beschrieben, worin R für $C_1$-$C_4$-Alkyl steht. Diese zeigen wertvolle mikrobizide Eigenschaften. Sie lassen sich zur Bekämpfung von pflanzenschädlichen Mikroorganismen, insbesondere gegen phytopathogene Pilze verwenden. So besitzen die Verbindungen der Formel I eine für praktische Bedürfnisse sehr günstige Wirkung zum Schutze von Kulturpflanzen, ohne diese durch unerwünschte Nebenwirkungen zu beeinflussen. Sie lassen sich in der Praxis in Form von Schädlingsbekämpfungsmittel anwenden. Es wird auch ein Verfahren zur Herstellung dieser Wirkstoffe und deren Formulierung zu mikrobiziden Mitteln und ihre Anwendung im Pflanzenschutz beschrieben.

EP 0 043 348 A1

CIBA-GEIGY AG
Basel (Schweiz)

5-12931/=

Imidazolidin-2,4-dion-Derivate, Verfahren zu deren Herstellung und deren Verwendung als Mikrobizide.

Die vorliegende Erfindung betrifft Verbindungen der Formel I

```
      O       O
Cl    ‖       ‖
 \•–•\   /C – N – CS – R
•//    •–N      |
 \•=•/   \C – CH2
 /        ‖
Cl        O
```

(I),

worin R $C_1$-$C_4$-Alkyl bedeutet.

Unter Alkyl oder als Alkylteil eines anderen Substituenten sind je nach Zahl der angegebenen Kohlenstoffatome folgende Gruppen zu verstehen: Methyl, Aethyl, Propyl und Butyl, sowie ihre Isomere, wie iso-Propyl, iso-Butyl, sek.-Butyl und tert.-Butyl.

Verbindungen der Formel I sind sehr wertvolle Wirkstoffe gegen pflanzenschädigende Mikroorganismen.

Die Verbindungen der Formel I können hergestellt werden, indem man

a) eine Verbindung der Formel II

```
      O
Cl    ‖
 \•–•\   /C – NH
•//    •–N      |
 \•=•/   \C – CH2
 /        ‖
Cl        O
```

(II)

in Gegenwart eines säurebindenden Mittels mit einer Verbindung der Formel III

```
 O
 ‖
HalCSR
```

(III)

umsetzt oder

b) eine Verbindung der Formel IV

(IV)

mit einer Verbindung der Formel V

MSR (V)

umsetzt, wobei in den Formeln II bis V R die für die Formel I gegebenen Bedeutungen hat und Hal für Halogen, vorzugsweise Chlor oder Brom und M für Wasserstoff oder ein Alkali- oder Erdalkalimetallatom stehen.

Wenn M = Wasserstoff ist, wird das Verfahren b) ebenfalls in Gegenwart eines säurebindenden Mittels durchgeführt. Es können bei beiden Verfahren grundsätzlich Lösungsmittel verwendet werden, die den Reaktionsteilnehmern gegenüber inert sind.

Beispiele solcher Lösungsmittel sind: Ester, wie Essigsäuremethylester, Essigsäureäthylester, Essigsäurebutylester; Aliphaten oder Aromaten wie Benzol, Toluol, Xylole, Petroläther; halogenierte Kohlenwasserstoffe wie Chlorbenzol, Methylenchlorid, Aethylenchlorid, Chloroform; Aether und ätherartige Verbindungen wie Dialkyläther, Dioxan, Tetrahydrofuran; Nitrile wie Acetonitril; Dimethylsulfoxid, Ketone wie Methyläthylketon und Gemische solcher Lösungsmittel untereinander.

Als säurebindende Mittel kommen z.B. tertiäre Amine wie Trialkylamine (z.B. Triäthylamin), Pyridin und Pyridinbasen wie 4-Dimethylaminopyridin oder 4-Pyrrolidylopyridin, oder anorganische Basen, wie die Oxide, Hydroxide, Hydrogencarbonate, Carbonate oder Hydride von Alkali- und Erdalkalimetallen in Betracht.

Die Umsetzungen erfolgen bei Temperaturen zwischen -40° bis 100°C, vorzugsweise -20° bis 40°C und bei Normaldruck.

Die beiden Verfahrensvarianten a und b gehören ebenfalls zur Erfindung.

Die Ausgangsstoffe werden nach an sich bekannten Methoden hergestellt.

Die Verbindung der Formel IV kann z.B. durch Umsetzung einer Verbindung der Formel II mit Phosgen gewonnen werden.

Methoden zur Herstellung der Verbindungen der Formel II sind z.B. in den DE-Offenlegungsschriften 1,958,183 und 2,636,549 beschrieben.

Die Verbindungen der Formel I können für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen, wie z.B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90%; der Gehalt an flüssigen und festen Zuschlagstoffen zwischen 10 und 99,9% sowie 0 bis 25% eines Tensides.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10%) Granulate, Umhüllungsgranulate, Imprägnierungsgranulate und Homogengranulate, Pellets (Körner) (1 bis 80%);

Flüssige Aufarbeitungsformen:

a) in Wasser dispergierbare Wirkstoffkonzentrate:
   Spritzpulver (wettable powders) und Pasten (25-90% in der Handelspackung, 0,01 bis 15% in gebrauchsfertiger Lösung); Emulsions- und Lösungskonzentrate (10 bis 50%; 0,01 bis 15% in gebrauchsfertiger Lösung);

b) Lösungen (0,1 bis 20%); Aerosole.

Solche Mittel gehören ebenfalls zur Erfindung.

Aehnliche Substanzen sind aus den DE-Offenlegungsschriften 1,958,183; 2,049,531; 2,149,923; 2,753,823; 2,636,549; 2,833,767 sowie aus der FR-Offenlegungsschrift 2,308,626 als Mikrobizide bekannt, zeigen aber den erfindungsgemässen Verbindungen gegenüber gewisse Nachteile.

Es wurde gefunden, dass Verbindungen der Formel I ein für die praktischen Bedürfnisse sehr günstiges Mikrobizid-Spektrum zum Schutze von Kulturpflanzen aufweisen und in den zur Bekämpfung von Mikroorganismen erforderlichen Konzentrationen keinen schädlichen Einfluss auf die Kulturpflanzen ausüben. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, vor allem aber Reben, Hopfen, Gurkengewächse (Gurken, Kürbis, Melonen), Solanaceen wie Kartoffeln, Tabak und Tomaten, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Eine besonders ausgeprägte fungizide Aktivität entfalten sie gegen parasitäre Pilze auf oberirdischen Pflanzenteilen. Die Wirkstoffe sind gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen die der Klasse der Fungi imperfecti (Deuteromycetes) angehörenden Gattungen wie z.B. Alternaria, Cercospora, Verticillium und bevorzugt Botrytis [so beispielsweise gegen den Erreger des Grauschimmels an Erdbeeren oder Trauben (Botrytis cinerea)] eingesetzt werden.

Sie können ferner als Beizmittel zur Behandlung von Saatgut (Früchte, Knollen, Körner) und Pflanzenstecklingen zum Schutz vor Pilzinfektionen sowie gegen im Erdboden auftretende Mikroorganismen eingesetzt werden.

Die Erfindung betrifft somit ferner die Verwendung der Verbindungen der Formel I zur Bekämpfung phytopathogener Mikroorganismen, insbesondere zur Bekämpfung oder Verhütung eines Befalls durch pflanzenschädigende Pilze.

Die Verbindungen der Formel I, worin R einen verzweigten $C_1$-$C_4$-Alkylrest darstellt und insbesondere die nachfolgenden Einzelsubstanzen, sind besonders bevorzugt aufgrund ihrer ausgeprägten Aktivität gegen phytopathogene Pilze, vor allem gegen Fungi imperfecti, insbesondere Botrytis-Arten.

3-(3,5-Dichlorphenyl)-1-tert.butylthiocarbonyl-2,4-dioxo-imidazolidin

3-(3,5-Dichlorphenyl)-1-iso.propylthiocarbonyl-2,4-dioxo-imidazolidin

3-(3,5-Dichlorphenyl)-1-methylthiocarbonyl-2,4-dioxo-imidazolidin.

Die nachfolgenden Beispiele dienen zur näheren Erläuterung der Erfindung, ohne dieselbe einzuschränken. Die Temperaturen sind in Celsiusgraden angegeben. Prozente und Teile beziehen sich auf Gewicht.

Herstellungsbeispiel

Beispiel 1: Herstellung von

```
 Cl          O           O
   \         ||          ||
   /=\      C ——— N - CSCH3
  <( )>—N<           |
   \=/      C ——— CH2
   /         ||
 Cl          O
```

3-(3,5-Dichlorphenyl)-1-methylthiocarbonyl-2,4-dioxo-imidazolidin.

Zu 24,5 g 3-(3,5-Dichlorphenyl)-imidazolidin-2,4-dion in 600 ml Essigsäureäthylester wurden unter Rühren bei Raumtemperatur innerhalb 1 Stunde gleichzeitig 12 g Thiokohlensäure-S-methylesterchlorid und 11 g Triäthylamin, gelöst in je 50 ml Essigsäureäthylester, zugetropft. Nach 12-stündigem Rühren bei Raumtemperatur wurde dreimal mit je 50 ml Wasser gewaschen, über Natriumsulfat getrocknet, filtriert und das Lösungsmittel abgedampft. Nach dem Umkristallisieren aus Toluol schmelzen die weissen Kristalle bei 180-185°.

Auf analoge Weise wurden folgende Verbindungen der Formel I hergestellt:

Tabelle 1

| Verb.Nr. | R | Physik. Daten [°C] |
|---|---|---|
| 1 | $-CH_3$ | Smp. 180 - 185 |
| 2 | $-C_2H_5$ | Smp. 124 - 127 |
| 3 | $-CH_2CH_2CH_3$ | Smp. 123 - 124 |
| 4 | $-CH(CH_3)-CH_3$ | Smp. 133 - 134 |
| 5 | $-CH(CH_3)-CH_2CH_3$ | Smp. 107 - 110 |
| 6 | $-CH_2-CH(CH_3)-CH_3$ | Smp. 129 - 130 |
| 7 | $-C(CH_3)_2-CH_3$ | Smp. 138 - 140 |

## Formulierungsbeispiele

### Beispiel 2

Stäubemittel: Zur Herstellung eines a) 5%igen und b) 2%igen Stäubemittels werden die folgenden Stoffe verwendet:

a) 5 Teile eines der Wirkstoffe Nr. 1 bis 7
95 Teile Talkum

b) 2 Teile eines der Wirkstoffe Nr. 1 bis 7
1 Teil hochdisperse Kieselsäure
97 Teile Talkum

Die Wirkstoffe werden mit den Trägerstoffen vermischt und vermahlen und können in dieser Form zur Anwendung verstäubt werden.

### Beispiel 3

Granulat: Zur Herstellung eines 5%igen Granulates werden die folgenden Stoffe verwendet:

5 Teile eines der Wirkstoffe Nr. 1 bis 7
0,25 Teile epoxidiertes Pflanzenöl
0,25 Teile Cetylpolyglykoläther
3,50 Teile Polyäthylenglykol
91 Teile Kaolin (Korngrösse 0,3 - 0,8 mm).

Die Aktivsubstanz wird mit epoxidiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 4

Spritzpulver: Zur Herstellung eines a) 70%igen, b) 40%igen, c) und d) 25%igen, e) 10%igen Spritzpulvers werden folgende Bestandteile verwendet:

a)
70 Teile eines der Wirkstoffe Nr. 1 bis 7
5 Teile Natriumdibutylnaphthylsulfonat
3 Teile Naphthalinsulfonsäuren-Phenolsulfonsäuren-Formaldehyd-Kondensat 3:2:1
10 Teile Kaolin
12 Teile Champagne-Kreide

b)
40 Teile eines der Wirkstoffe Nr. 1 bis 7
5 Teile Ligninsulfonsäure-Natriumsalz
1 Teil Dibutylnaphthalinsulfonsäure-Natriumsalz
54 Teile Kieselsäure

c)
25 Teile eines der Wirkstoffe Nr. 1 bis 7
4,5 Teile Calcium-Ligninsulfonat
1,9 Teile Champagne-Kreide/Hydroxyäthylcellulose-Gemisch 1:1
1,5 Teile Natrium-dibutyl-naphthalinsulfonat
10,5 Teile Kieselsäure
19,5 Teile Champagne-Kreide
28,1 Teile Kaolin

d)
25 Teile eines der Wirkstoffe Nr. 1 bis 7
2,5 Teile Isooctylphenoxy-polyoxyäthylen-äthanol
1,7 Teile Champagne-Kreide/Hydroxyäthylcellulose-Gemisch 1:1
8,3 Teile Natriumaluminiumsilikat
16,5 Teile Kieselgur
46 Teile Kaolin

e)
10 Teile eines der Wirkstoffe Nr. 1 bis 7
3 Teile Gemisch der Natriumsalze von gesättigten Fettalkoholsulfaten
5 Teile Naphthalinsulfonsäure/Formaldehyd-Kondensat
82 Teile Kaolin

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen.

Beispiel 5

Emulgierbare Konzentrate: Zur Herstellung eines 25%igen emulgierbaren Konzentrates werden folgende Stoffe verwendet:

25 Teile eines der Wirkstoffe Nr. 1 bis 7
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol

Aus solchen Konzentrationen können durch Verdünnen mit Wasser Emulsionen der gewünschten Anwendungskonzentration hergestellt werden, die besonders zur Blattapplikation geeignet sind.
Derartige Mittel und deren Herstellung sind Bestandteil dieser Erfindung.

Biologische Beispiele

Beispiel 6

Wirkung gegen Puccinia graminis auf Weizen

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit und ca. 20° wurden die infizierten Pflanzen in einem Gewächshaus bei ca. 22° aufgestellt.
Die Beurteilung der Rostpustelnentwicklung erfolgte 12 Tage nach der Infektion, hierbei zeigten die Verbindungen der Formel I eine gute Wirkung.

Die Verbindungen Nr. 1 und 7 unterdrückten den Puccinia-Befall sogar vollständig (0 bis 5%).

Beispiel 7

Wirkung gegen Botrytis auf Puffbohnen

Ca. 10 cm hohe Puffbohnenpflanzen wurden mit einer aus Spritzpulver des Wirkstoffes hergestellten Spritzbrühe (0,02% Aktivsubstanz) besprüht. Nach 24 Stunden wurden die behandelten Pflanzen mit einer Konidiensuspension des Pilzes infiziert. Nach einer Inkubation der infizierten Pflanzen während 2-3 Tagen bei 95-100% relativer Luftfeuchtigkeit und 21° erfolgte die Beurteilung des Pilzbefalles. Im Vergleich zu unbehandelten Kontrollpflanzen (=100% Befall) zeigten die Pflanzen, die mit einem der Wirkstoffe 1 bis 7 behandelt wurden einen Pilzbefall von weniger als 20%. Die Verbindungen Nr. 1, 4 und 7 unterdrückten den Befall auf weniger als 5%, Verbindung Nr. 7 sogar vollständig (0%).

Beispiel 8

Wirkung gegen Rhizoctonia solani auf Baumwolle

Der Pilz wurde auf sterilen Hirsekörnern kultiviert und einer Erde-Sand-Mischung beigegeben. Die infizierte Erde wurde in Schalen abgefüllt und mit Baumwollsamen besät. Gleich nach der Aussaat wurde das als Spritzpulver formulierte Versuchspräparat als wässerige Suspension über die Erde gegossen (20 ppm Aktivsubstanz bezogen auf das Erdvolumen). Die Erdschalen wurden darauf während 2-3 Wochen bei ca. 24° im Gewächshaus aufgestellt und durch leichtes Ueberbrausen stets gleichmässig feucht gehalten. Bei der Auswertung der Tests wurde der Auflauf der Baumwollpflanzen bestimmt. Nach Behandlung mit Spritzpulvern, die als Aktivsubstanz eine der Verbindungen Nr. 3, 5, 6 oder 7 enthielten, liefen über 80% der Baumwollsamen auf, und die Pflanzen hatten ein gesundes Aussehen.

Beispiel 9

Wirkung gegen Fusarium nivale auf Weizen

Weizenkörner wurden mit einer Sporensuspension des Pilzes kontaminiert und wieder angetrocknet. Die kontaminierten Körner wurden mit einer aus Spritzpulver hergestellten Suspension der Testsubstanz gebeizt (600 ppm Wirkstoff bezogen auf das Gewicht der Samen). Nach zwei Tagen wurden die Körner auf geeignete Agarschalen ausgelegt und nach weiteren vier Tagen wurde die Entwicklung der Pilzkolonien um die Körner herum beurteilt. Anzahl und Grösse der Pilzkolonien wurden zur Beurteilung der Testprodukte herangezogen.

Verbindungen der Formel I zeigten hierbei eine gute fungizide Wirksamkeit, insbesondere Verbindung Nr. 2 unterdrückte die Entwicklung von Pilzkolonien vollständig (0 bis 5%).

Patentansprüche: (für alle benannten Länder ausser Oesterreich)

1. Verbindungen der Formel I

$$\text{3,5-}Cl_2C_6H_3-N\left\langle\begin{array}{l} C(=O)-N-CS(=O)-R \\ \quad\quad\quad\;\; | \\ C(=O)-CH_2 \end{array}\right. \quad (I),$$

worin R $C_1$-$C_4$-Alkyl bedeutet.

2. Die Verbindung 3-(3,5-Dichlorphenyl)-1-tert.butylthiocarbonyl-2,4-dioxo-imidazolidin.

3. Die Verbindung 3-(3,5-Dichlorphenyl)-1-iso.propylthiocarbonyl-2,4-dioxo-imidazolidin.

4. Die Verbindung 3-(3,5-Dichlorphenyl)-1-methylthiocarbonyl-2,4-dioxo-imidazolidin.

5. Verfahren zur Herstellung einer Verbindung gemäss Anspruch 1, dadurch gekennzeichnet, dass man
a) eine Verbindung der Formel II

$$\text{3,5-}Cl_2C_6H_3-N\left\langle\begin{array}{l} C(=O)-NH \\ \quad\quad\quad\; | \\ C(=O)-CH_2 \end{array}\right. \quad (II)$$

in Gegenwart eines säurebindenden Mittels mit einer Verbindung der Formel III

$$\text{HalC(=O)SR} \quad (III)$$

umsetzt oder

b) eine Verbindung der Formel IV

$$\text{2,6-}Cl_2C_6H_3-N\langle\begin{matrix}C(=O)-NCOCl \\ | \\ C(=O)-CH_2\end{matrix} \quad \text{(IV)}$$

mit einer Verbindung der Formel V

MSR (V)

umsetzt, wobei in den Formeln III und V der Substituent R die unter Formel I angegebenen Bedeutungen hat und Hal für Halogen und M für Wasserstoff oder ein Alkali- oder Erdalkalimetallatom stehen.

6. Verfahren nach Anspruch 5, dadurch gekennzeichnet, dass man die Umsetzung in einem reaktionsinerten Lösungsmittel durchführt.

7. Verfahren nach Anspruch 5, dadurch gekennzeichnet, dass man die Umsetzung bei Temperaturen von -40° bis +100° C durchführt.

8. Verfahren nach Anspruch 7, dadurch gekennzeichnet, dass man die Umsetzung bei Temperaturen von -20° bis +40° C durchführt.

9. Verfahren nach einem der Ansprüche 5 bis 8, dadurch gekennzeichnet, dass der Substituent Hal in Formel III für Chlor oder Brom steht.

10. Mittel zur Bekämpfung und/oder präventiven Verhütung eines Befalls durch phytopathogene Mikroorganismen, dadurch gekennzeichnet, dass es als mindestens eine aktive Komponente eine Verbindung der Formel I nach Anspruch 1 enthält.

11. Mittel nach Anspruch 10, dadurch gekennzeichnet, dass es als aktive Komponente eine Verbindung der Formel I gemäss einem der Ansprüche 2 bis 4 enthält.

12. Mittel nach einem der Ansprüche 10 oder 11, dadurch gekennzeichnet, dass es 0,1 bis 90 Gewichtsprozent des Wirkstoffs, 10 bis 99,9 Gewichtsprozent an flüssigen oder festen Zuschlagstoffen sowie 0 bis 25 Gewichtsprozent eines Tensides enthält.

13. Verwendung einer Verbindung der Formel I nach Anspruch 1 zur Bekämpfung und/oder präventiver Verhütung eines Befalls von Pflanzen durch phytopathogene Mikroorganismen.

14. Verwendung nach Anspruch 13 von Verbindungen der Formel I gemäss einem der Ansprüche 2 bis 4.

15. Verwendung nach einem der Ansprüche 13 oder 14 dadurch gekennzeichnet, dass es sich bei den Mikroorganismen um phytopathogene Pilze handelt.

16. Verwendung nach Anspruch 15 zur Bekämpfung von Botrytis-Pilzen.

Patentansprüche (für Oesterreich)

1. Mikrobizides Mittel zur Bekämpfung und/oder Verhütung eines Befalls durch phytopathogene Mikroorganismen, dadurch gekennzeichnet, dass es als mindestens eine aktive Komponente eine Verbindung der Formel I enthält,

```
               O      O
  Cl           ||     ||
    \•--•    ,C - N - CS - R
   //    \\•-N'      |            (I),
  •\     /    \C - CH2
    \•==•      ||
  Cl/          O
```

worin R $C_1$-$C_4$-Alkyl bedeutet.

2. Mittel nach Anspruch 1, dadurch gekennzeichnet, dass es 0,1 bis 90 Gewichtsprozent des Wirkstoffs, zwischen 10 und 99,9 Gewichtsprozent an flüssigen und festen Zuschlagstoffen sowie 0 bis 25 Gewichtsprozent eines Tensides enthält.

3. Mittel nach einem der Ansprüche 1 oder 2, dadurch gekennzeichnet, dass es als Wirkstoff die Verbindung 3-(3,5-Dichlorphenyl)-1-tert. butylthiocarbonyl-2,4-dioxo-imidazolidin enthält.

4. Mittel nach einem der Ansprüche 1 oder 2, dadurch gekennzeichnet, dass es als Wirkstoff die Verbindung 3-(3,5-Dichlorphenyl)-1-iso. propylthiocarbonyl-2,4-dioxo-imidazolidin enthält.

5. Mittel nach einem der Ansprüche 1 oder 2, dadurch gekennzeichnet, dass es als Wirkstoff die Verbindung 3-(3,5-Dichlorphenyl)-1-methylthiocarbonyl-2,4-dioxo-imidazolidin enthält.

6. Verwendung eines Mittels nach einem der Ansprüche 1 bis 6 zur Bekämpfung phytopathogener Pilze.

7. Verwendung nach Anspruch 6, dadurch gekennzeichnet, dass es sich bei den Pilzen um die Gattung Botrytis handelt.

8. Verfahren zur Herstellung von Verbindungen gemäss Anspruch 1, dadurch gekennzeichnet, dass man

a) eine Verbindung der Formel II

```
                    O
  Cl                ‖
    \•-•           C - NH
   //   \\        /    |
  •       •-N          |          (II)
   \     /        \    |
    •=•            C - CH2
   /               ‖
  Cl               O
```

in Gegenwart eines säurebindenden Mittels mit einer Verbindung der Formel III

```
        O
        ‖
      HalCSR              (III)
```

umsetzt oder

b) eine Verbindung der Formel IV

```
                    O
  Cl                ‖
    \•-•           C - NCOCl
   //   \\        /    |
  •       •-N          |          (IV)
   \     /        \    |
    •=•            C - CH2
   /               ‖
  Cl               O
```

mit einer Verbindung der Formel V

$$MSR \qquad (V)$$

umsetzt, wobei in den Formeln III und V der Substituent R die für Formel I angegebenen Bedeutungen hat, Hal für Halogen und M für Wasser-

stoff, ein Alkali- oder Erdalkalimetallatom stehen.

9. Verfahren nach Anspruch 8, dadurch gekennzeichnet, dass man die Umsetzung in einem reaktionsinerten Lösungsmittel durchführt.

10. Verfahren nach einem der Ansprüche 8 oder 9, dadurch gekennzeichnet, dass man die Umsetzung bei Temperaturen von -40° bis +100°C durchführt.

11. Verfahren nach Anspruch 10, dadurch gekennzeichnet, dass man die Umsetzung bei Temperaturen von -20° bis +40°C durchführt.

12. Verfahren nach einem der Ansprüche 8 bis 11, dadurch gekennzeichnet, dass der Substituent Hal in Formel III für Chlor oder Brom steht.

13. Verfahren nach einem der Ansprüche 8 bis 12, dadurch gekennzeichnet, dass man eine Verbindung der Formel I gemäss einem der Ansprüche 3 bis 5 herstellt.

Europäisches Patentamt

# EUROPÄISCHER RECHERCHENBERICHT

**0043348**

Nummer der Anmeldung

EP 81 81 0259

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 2 149 923 (RHONE-POULENC)<br>* Seiten 1 und 2 *<br>-- | 1,5,10 |
| A | CHEMICAL ABSTRACTS, Band 87, Nr. 9, 29. August 1977, Zusammenfassung Nr. 68361v, Seite 554 COLUMBUS OHIO (US)<br>& JP - A - 77 33 673 (NIPPON SODA CO., LTD.) (14.03.1977)<br>----- | 1,5,10 |

KLASSIFIKATION DER ANMELDUNG (Int. Cl.³)

C 07 D 233/80
A 01 N 43/50

RECHERCHIERTE SACHGEBIETE (Int. Cl.³)

C 07 D 233/80
A 01 N 43/50

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 01.09.1981 | DE BUYSER |